Europäisches Patentamt

European Patent Office

Office européen des brevets

(11) Publication number: **0 393 099 B1**

(12) **EUROPEAN PATENT SPECIFICATION**

(45) Date of publication of patent specification: **23.02.94**

(51) Int. Cl.⁵: **C07C 5/333**, B01J 23/08, B01J 23/14, B01J 29/32, B01J 29/34

(21) Application number: **88910354.5**

(22) Date of filing: **04.11.88**

(86) International application number:
**PCT/US88/03944**

(87) International publication number:
**WO 89/04818 (01.06.89 89/12)**

(54) **A DEHYDROGENATION CATALYSTSYNTHESIS AND ITS USE.**

(30) Priority: **17.11.87 US 122089**
**28.12.87 US 138430**
**28.12.87 US 138462**
**28.12.87 US 138463**
**28.12.87 US 138471**
**28.12.87 US 138472**
**28.12.87 US 138473**
**28.12.87 US 138474**
**24.06.88 US 210946**
**24.06.88 US 210947**
**24.06.88 US 210948**
**24.06.88 US 211198**
**24.06.88 US 211207**

(43) Date of publication of application:
**24.10.90 Bulletin 90/43**

(45) Publication of the grant of the patent:
**23.02.94 Bulletin 94/08**

(84) Designated Contracting States:
**BE DE FR GB IT NL SE**

(73) Proprietor: **MOBIL OIL CORPORATION**
**150 East 42nd Street**
**New York New York 10017(US)**

(72) Inventor: **CHEN, Nai, Yuen**
**4 Forest Central Drive**
**R.D. Titusville, NJ 08560(US)**
Inventor: **DESSAU, Ralph, Moritz**
**14 Celler Road**
**Edison, NJ 08817(US)**
Inventor: **PARTRIDGE, Randall, David**
**58 Jacobs Creek Road**
**West Trenton, NJ 08628(US)**
Inventor: **VALYOCSIK, Ernest, William**
**960 Randolph Drive**
**Yardley, PA 19067(US)**
Inventor: **VARTULI, James, Clarke**
**320 Ponds Edge Road**
**West Chester, PA 19380(US)**
Inventor: **VENKAT, Chaya, Rao**
**35 Littlebrook Road North**
**Princeton, NJ 08540(US)**

(56) References cited:

| | |
|---|---|
| **EP-A- 0 018 498** | **EP-A- 0 232 182** |
| **US-A- 3 878 131** | **US-A- 4 049 539** |
| **US-A- 4 430 517** | **US-A- 4 486 547** |
| **US-A- 4 551 574** | **US-A- 4 614 834** |
| **US-A- 4 722 780** | **US-A- 4 737 262** |
| **US-A- 4 741 820** | |

(74) Representative: **Colmer, Stephen Gary et al**
**Patent Department**
**c/o Mobil Services Company Limited**
**Mobil Court**
**3 Clements Inn**
**London WC2A 2EB (GB)**

## Description

This invention relates to a dehydrogenation and dehydrocyclization catalyst, its synthesis and use.

Dehydrogenation/dehydrocyclization is a well known process used, for example, in the reforming of petroleum naphtha fractions. Thus, U.S. Patent No. 4,614,834 discloses a reforming catalyst comprising a non-acidic large pore zeolite, a Group VIII metal component and a silica support matrix derived by a high pH gelation of an alkaline metal silicate sol. The large pore zeolites disclosed are zeolite L, zeolite X, zeolite Y, and mordenite and the catalyst can optionally contain a series of catalyst modifying components, including rhenium, tin, cobalt, indium, gallium, lead, zinc, uranium, thallium, dysprosium, and germanium.

It has now been found that non-acidic ZSM-5 containing a dehydrogenation metal and selected metal modifiers exhibits high selectivity for dehydrogenation and/or dehydrocyclization of paraffins, together with decreased selectivity for hydrogenolysis (especially methane formation) and decreased aging, even in the absence of hydrogen.

Accordingly, the invention resides in a catalyst composition comprising a dehydrogenation metal and non-acidic ZSM-5 containing indium, tin, thallium, or lead.

The amount of dehydrogenation metal in the component can range from 0.01 to 30 wt% and preferably, from 0.1 to 10 wt% of the catalyst. In a preferred embodiment, platinum is the dehydrogenation metal. However, the dehydrogenation metal can be any Group VIII metal including those of the platinum group (namely, platinum, palladium, osmium, ruthenium, iridium, and rhodium), chromium and vanadium.

The catalyst of the invention also contains a metal modifier selected from indium, tin, lead, and thallium, which is present in an amount ranging from 0.01 to 20 wt%, preferably from 0.1 to 10 wt% of the catalyst.

Preferably, at least part of the modifier present forms part of the framework of the ZSM-5.

The ZSM-5 employed in the catalyst of the invention may have a silica/alumina molar ratio up to 1000, or even greater, and preferably contains less than 0.1 wt% aluminum. Moreover, the zeolite may contain other elements than the metal modifiers and its basic framework elements silica and aluminum, such as boron, iron, chromium, and gallium. The content of these other elements in the zeolite can range from 0 to 10 wt%.

Preferably at least some of the dehydrogenation metal is intrazeolitic, that is, some of that metal is within the pore structure of the ZSM-5, although some of the metal may be on the surface of the zeolite. A test for determining whether, for example, Pt is intrazeolitic or extrazeolitic in the case of ZSM-5 is reported by R.M. Dessau, J. CATAL. Vol. 89, p. 520 (1984). The test is based on the selective hydrogenation of olefins.

The catalyst composition of the invention comprising dehydrogenation metal combined with a metal-modified ZSM-5 is characterized by a lack of any appreciable acid activity. This catalyst therfore meets the criteria of non-acidic catalysts described by Davis and Venuto, J. CATAL. Vol. 15, p. 363 (1969). Thus, a non-equilibrium mixture of xylenes are formed from either n-octane or each individual methylheptane isomer, with the n-octane yielding mosstly o-xylene and 2-methyl-heptane yeilding mostly m-xylene, at conversions between 10 and 60%. An additional method of testing the non-acid character of the catlyst composition of the invention is to add 100 mg of the catalyst to 30 ml distilled deionized water (with a pH of 7) maintained under an inert atmosphere (i.e., free of $CO_2$), such as an argon atmosphere, whereby with the non-acidic composition of the invention the water will have a pH of at least 6 and preferably greater than 7.

When used to effect dehydrogenation of paraffins, for example, to aromatics, the catalyst compositions of the invention decrease the hydrogen content of the feed to produce a product having the same number of carbon atoms as the feed. In contrast, equivalent catalyst compositions free of the modifier also catalyze hydrogenolysis of paraffins, e.g., to methane, as a major competing side reaction; and, accordingly, the latter compositions exhibit decreased selectivity for the aromatization of paraffins but increased selectivity for $C_1$ - $C_5$ paraffin production. Some of the aforementioned catalysts were screened for heptane aromatization at 538°C in the presence of nitrogen diluent. The results are shown in Table A below.

Table A

| Paraffin Aromatization over Pt/ZSM-5 | | | | |
|---|---|---|---|---|
| Modifier | % Conversion | Toluene Sel | Benzene Sel | CH$_4$ Sel |
| Sn | 99.3 | 95.0% | 1.5% | 0.4% |
| In | 98.2 | 92.7% | 1.8% | 0.5% |
| Pb | 98.7 | 95.4% | 1.1% | 0.4% |
| Tl | 99.6 | 85.7% | 6.7% | 1.7% |
| None | 96.3 | 40.9% | 19.4% | 9.3% |
| B | 94.7 | 30.2% | 32.8% | 20.7% |
| Cr | 95.5 | 44.4% | 20.4% | 3.4% |
| Ti | 96.1 | 31.8% | 32.6% | 19.7% |
| Sc | 96.3 | 38.9% | 40.6% | 16.0% |
| Au | 90.7 | 21.1% | 45.1% | 20.8% |
| Ni | 94.3 | 42.4.% | 19.7% | 7.2% |
| Ge | 96.3 | 47.0% | 19.9% | 6.6% |
| Zr(470°C) | 96.8 | 49.0% | 16.3% | 7.9% |

The reactions summarized in Table A were conducted at 30 torr (4kPa) n-heptane in N$_2$ at 538°C and 1 atm (100 kPa) total pressure and selectivities are given on H$_2$-free weight basis.

The non-acidic platinum catalyst prepared from ZSM-5 modifed with In, Sn, Pb, and Tl provided much higher aromatics selectivity than all the other catalysts examined. Toluene selectivity from heptane was greater than 85% at 99% conversion (H$_2$ free carbon basis). The other catalysts, including Pt/B-ZSM-5 and Pt/high silica:alumina ratio ZSM-5, also exhibited no appreciable acid activity so that platinum chemistry dominated. However with these other catalysts, although significant metal-catalyzed aromatization was observed, hydrogenolysis to methane constituted a major competing side reaction. The highest toluene selectivity from n-heptane observed was 50%, and in most cases that selectivity was significantly lower.

The metal modified crystalline materials of the invention can be made in various ways and, for convenience, the ensuing description will refer to production of indium-containing materials. Similar techniques can, however, be employed to produce materials containing tin, lead, and/or thallium.

Indium incorporation can be during synthesis or post-synthesis; and the materials can be prepared either by stepwise or simultaneous incorporation of the indium and the hydrogenation/dehydrogenation function to the crystallization reaction product. The dehydrogenation function can be first introduced to the synthesis product with subsequent indium incorporation, or vice versa. Stepwise preparation includes techniques of cocrystallization (by inclusion of an indium compound in the syntehsis mixture used to produce the crystalline material), impregnation, or ion exchange. Simultaneous incorporation includes the combination of indium with the dehydrogenation/hydrogenation function during synthesis (i.e., crystallization) or simultaneously after synthesis of the crystalline material.

An indium free material can be treated with indium compounds at elevated temperatures. Such treatments can be conducted so that the source of indium is either in the gaseous phase (such as indium chloride) or the liquid phase including the aqueous phase (such as indium nitrate). Alternatively, an indium free crystalline reactant can simply be impregnated with an indium source and then calcined at temperatures above 400°C.

In the materials of the invention, all cation-exchangeable sites are occupied by cations other than hydrogen and other than hydrogen precursors, such as NH$_4{}^+$. Specifically, such sites are occupied by Na$^+$, K$^+$, Cs$^+$, Ca$^+$, Mg$^{++}$, Ba$^{++}$, Sr$^{++}$, or admixtures thereof, although some sites may of course be occupied by the metal modifier or hydrogenation/dehydrogenation metal. The alkali metals serve to neutralize any acidity due to framework aluminum. The source of alkali metal cation can derive from cations incorporated during synthesis, in excess of the aluminum content thereof. Alternatively, one can treat the final product with a basic solution of an alkali metal hydroxide as a final step prior to use, as described for example in U.S. Patent No. 4,652,360.

The metal modifier and dehydrogenation metal containing materials of the invention can be combined with a matrix or binder material to render them attrition resistant and more resistant to the severity of the conditions to which they will be exposed during use in hydrocarbon conversion applications. The combined compositions can contain 1 to 99 weight percent of the materials of the invention based on the combined weight of the matrix (binder) and material of the invention. When used in dehydrogenation and/or dehydrocyclization, the material of the invention will preferably be combined with non-acidic matrix or binder materials, such as oxides of Groups IVA and IVB of the Periodic Table, most preferably silica.

The catalyst composition of the invention is useful for the dehydrogenation of hydrocarbons having at least 2 carbon atoms, and in particular for dehydrocyclization of aliphatics containing at least 6 carbon atoms. Examples of such reactions are discussed below.

Reforming

Catalytic reforming is a well known process in which hydrocarbon molecules are rearranged, or reformed in the presence of a catalyst. The molecular rearrangement results in an increase in the octane rating of the feedstock. Thus, during reforming low octane hydrocarbons in the gasoline boiling range are converted into high octane components by dehydrogenation of naphthenes and isomerization, dehydrocyclization and hydrocracking of paraffins.

When reforming is undertaken over the catalyst composition of the invention, the conditions employed generally include a termperature of 427 to 595°C (800 to 100°F), preferably 482 to 566°C (900 to 1050°F); a pressure of 100 - 3550 kPa (1 atmosphere to 500 psig), preferably from 300 to 1825 kPa (30 psig to 250 psig); an inlet $H_2$/hydrocarbon of 20 or less, even zero as discussed in the Examples (because of hydrogen production during reforming, there will be a hydrogen partial pressure in the unit); and an LHSV (liquid hourly space velocity) of 0.1 to 20, preferably 0.1 to 10.

The feedstock reformed by the catalyst composition of the invention can be a straight-run thermal or catalytically cracked naphtha, conveniently having a boiling range of 65 to 205°C (180 to 250°F) so as to contain $nC_6$ + paraffins. Preferably, the feedstock is a light naphtha fraction boiling at 80 to 120°C (180 to 250°F) so as to contain $nC_6$ - $C_7$ paraffins. Because of competing isomerization and hydrocracking reactions, such light naphtha fractions are difficult to convert selectively to aromatics over conventional reforming catalyst. Prior to reforming, the naphtha feedstock may be hydrotreated in conventional manner to reduce sulfur and/or nitrogen contaminants.

The naphtha feedstock may also be cofed with a non-hydrogen diluent which is inert to aromatization under the reforming conditions and which thereby reduces the hydrogen partial pressure in the reforming reactor. Suitable diluents include helium, nitrogen, carbon dioxide, and light hydrocarbons through $C_5$ such as methane, ethane, propane, butane, pentane, ethylene, propylene, butenes, pentenes and mixtures thereof. The use of $C_3$ - $C_5$ hydrocarbons as cofeeds may be particularly desirable in that they can be easily separated from the hydrogen produced in the aromatization reactions. The diluent may also be recycle or part or all of the aromatic rich reformate. Accordingly, the diluents can constitute aromatic compounds. The diluent to hydrocarbon feed molar ratio can range from 1 to 20 with best results being obtained in the range of 2:1 to 10:1.

Post-reforming

Reformates having a research octane of 50 - 90, preferably 70 - 90, and containing significant quantities of $C_6$ and $C_7$ paraffins, such as are produced by many conventional reforming processes, are conveniently upgraded by contacting with the catalyst composition of the invention. Such post-reforming is found to increase the research octane and aromatics content to the reformate. Conditions for post-reforming are conviently the same as those described above for the reforming process of the invention.

Production of Benzene and/or Toluene

The catalyst composition of the invention, can be used to convert normal hexane and/or normal heptane to benzene and/or toluene. Suitable conditions for this process are:

|  | Temperature | WHSV ($C_6$-$C_7$) | Total Pressure |
|---|---|---|---|
| Broad | 400° to 600°C | 0.1 to 10.0 | 5 to 500 psia (30-3450 kpa) |
| Preferred | 450° to 550°C | 0.3 to 2.5 | 15 to 150 psia (100-1030 kPa) |

The n-hexane and/or n-heptane feed should be substantially "isomer-free", that is in the case n-hexane, the feed should be free of 2 and 3-methylpentane and 2,2 and 2,3-dimethylbutane. Similarly, the feed should be substantially free of cyclic compounds, such as methyl cyclopentane, since the presence of significant quantities of these materials is found to increase catalyst aging.

5

Substantially isomer-free normal hexane or normal heptane is obtainable from a distillation cut of natural gasoline. Suitable methods for separating the normal paraffins include superfractionation, urea adduction, and bulk separation by Type 5A molecular sieve. The preferred method is molecular sieve separation. Several such processes are in commercial use for the recovery of normal paraffins from refinery streams. During the adsorption step of such separation, the effluent contains isoparaffins and cyclic hydrocarbons. High purity normal paraffins are recovered by desorption or by displacement with a lighter normal paraffin such as propane. For purposes of the present invention, not more than 5 weight %, and preferably not more than 2.5 weight %, of isoparaffins and cyclic compounds should be present in the feed. The normal hexane, heptane, or mixture thereof should constitute at least 90 weight % of the total $C_5$ + hydrocarbons in the feed, and preferably 95 weight %.

In one embodiment of this invention, the normal hexane, heptane or mixture thereof is recovered by Type 5A molecular sieve separation using propane as desorbent, and the desorbed effluent containing propane is catalytically converted without prior separation of propane. Reduction of the partial pressure of the benzene and/or toluene product by propane favors its formation. Thus, in addition to the normal paraffins, the feed may contain a diluent such as hydrogen, an inert gas such as nitrogen, or an aliphatic hydrocarbon containing less than five carbon atoms. Although the conversion takes place with high selectivity and slow catalyst aging even in the absence of added hydrogen, the presence of a small amount of hydrogen may be used to further reduce aging. When hydrogen is used, it is preferred that it be used in conjunction with an inert gas or diluent described above.

Production of Sstyrene

The catalyst composition of the invention can also be used to convert n-octane to styrene, conveniently at a temperature of at least 500 °C in the presence of an inert gas diluent such as nitrogen.

Dehydrogenation of Hydrocarbons Containing $C_2$-$C_5$ Aliphatic Moities

$C_2$ - $C_5$ hydrocarbons or moities can be dehydrogenated to produce their unsaturated analogues using the catalyst-composition of the invention. Thus, one suitable class of reactants includes alkanes of 2 to 5 carbon atoms including ethane, propane, butane, isobutane, pentane, and 2-methylbutane. On dehydrogenation, these will yield ethylene, propylene, butene, isobutene, pentene, and isopentene, respectively. Another class of reactants includes olefins of 2 to 5 carbon atoms such as ethylene, butene, pentene, and isopentene. Dehydrogenation of ethylene will produce acetylene; dehydrogenation of butene will produce butadiene and dehydrogenation of methyl butene will produce isoprene.

A further class of reactants includes aryl and alkylaryl substituted aliphatics. Preferably, the alkyl group of the alkylaryl substituted aliphatic contains 1 - 4 carbon atoms. The aryl substituted aliphatic reactants embrace unsubstituted arylaliphatics and alkyl substituted aryl aliphatics and, similarly, each of the alkyls of said alkyl substituted alkylaryls contains preferably less than 4 carbon atoms. By way of illustration reactants such as ethylbenzene, diethylbenzene, ethyl toluene, and cumene are representative of these compounds. On dehydrogenation, ethyl benzene will produce styrene; p-ethyltoluene will produce p-methylstyrene; cumene, isopropenylbenzene; and diethylbenzene, divinylbenzene.

In accordance with the invention, catalytic dehydrogenation conditions include a pressure of 10 to 3550 kPa (0.1 atmosphere to 500 psig), a temperature of 300 °C to 700 °C, preferably 300 °C to 600 °C and most preferably from 400 °C to 600 °C, a reactor inlet $H_2$/feed ratios of 5 or less (even at reactor inlet ratios of zero (0), there will be a hydrogen partial pressure in the reactor because hydrogen is a bi-product of dehydrogenation); and a liquid hourly space velocity of 0.1 to 50, preferably 0.5 to 10.

Dewaxing

The catalyst composition of the invention may also be employed to dewax hydrocarbon feedstocks containing $C_{15}$ paraffins, whereby the latter are converted to distillate range products so that the pour point and wax content of the feed is reduced. Typical waxy feedstocks which can be treated includes those boiling in the range 180 to 550 °C (350 to 1025 °F) and having a pour point greater than -1 °C (+30 °F), such as gas oils, kerosenes, vacuum gas oils, whole crudes and oils derived from tar sands, shale and coal.

Typical dewaxing conditions are listed below.

| Pressure, broad | 0 - 100 psig (100 - 700 kPa) |
|---|---|
| Pressure, preferred | 20 - 500 psig (240 - 3550 kPa) |
| Temperature, broad | 500 - 1200 °F (260 - 650 °C) |
| Temperature, preferred | 800 - 1050 °F (430 - 565 °C) |
| WHSV | 0.1 - 20 |
| WHSV, preferred | 0.2 - 10 |
| $H_2$:oil | 0 - 20:1 |

The invention will now be more particularly described with reference to the following Examples and the accompanying drawings, in which:

Figure 1 is an X-ray diffraction pattern of In-ZSM-5 produced in Run No. 6 of Example 1;

Figure 2 is a graph plotting $C_5$ + yield in weight % against $C_5$ + RON value for reforming according to Example 13 compared to reforming using a conventional chloride $Pt/Al_2O_3$ ctalyst;

Figure 3 is a graph plotting gas chromatographic analysis of benzene and toluene (GC area %) against hours on stream during naphtha reforming for the Pt/Sn ZSM-5 catalyst of example 28.

## EXAMPLES

## EXAMPLE 1

Crystalline indium-containing ZSM-5 samples were produced according to the processes summarized in Table 1. It is to be noted that the products were synthesized from reaction mixtures containing no deliberately added sources of $Al_2O_3$.

## TABLE 1
### Crystallizations of Indium-Containing Zeolites
### 160°C; Stirred 400 rpm

Mixture Composition (Mole Ratios)

| Run No. | $SiO_2/In_2O_3$ | $H_2O/SiO_2$ | $OH^-/SiO_2$ | $Na^+/SiO_2$ | $R/SiO_2$ | Time, Days | Zeolite Product |
|---|---|---|---|---|---|---|---|
| 1a | 500 | 48 | 0.26 | 0.27 | 0.10[c] | 3 | ZSM-5 |
| 2b | 500 | 48 | 0.26 | 0.27 | 0.10[c] | 3 | ZSM-5 |
| 3a | 300 | 48 | 0.26 | 0.28 | 0.10[c] | 3 | ZSM-5 |
| 4b | 300 | 48 | 0.26 | 0.28 | 0.10[c] | 1 | ZSM-5 |
| 5d | 300 | 48 | 0.26 | 0.28 | 0.20[b] | 1 | ZSM-5 |
| 6b | 150 | 48 | 0.26 | 0.31 | 0.10[c] | 2 | ZSM-5 |
| 7b | 150 | 48 | 0.26 | 0.31 | 0.10[c] | 2 | ZSM-5 |
| 8b | 150 | 48 | 0.26 | 0.31 | 0.10[c] | 2 | ZSM-5 |
| 9b | 150 | 48 | 0.26 | 0.31 | 0.10[c] | 3 | ZSM-5 |
| 10e | 150 | 48 | 0.26 | 0.59 | 0.10[c] | 2 | ZSM-5 |
| 11f | 76 | 48 | 0.26 | 0.23 | 0.10[c] | 6 | ZSM-5 |
| 12b | 70 | 40 | 0.20 | 0.37 | 0.10[c] | 3 | ZSM-5 |
| 13a | 70 | 40 | 0.26 | 0.37 | 0.10[c] | 3 | ZSM-5 |
| 14a | 60 | 48 | 0.26 | 0.39 | 0.10 | 3 | ZSM-5 |

a -- Silica source is tetraethylorthosilicate $(Et_4SiO_4)$
b -- Silica source is SPEX Industries precipitated $SiO_2$
c -- R = TPA+
d -- Silica source is DeGussa fumed $SiO_2$
e -- Silica source is Q-brand sodium silicate
f -- Silica source is kieselsaure precipitated $SiO_2$

Table 2 is a compilation of chemical analyses of some of the indium-containing products. These products vary in indium content from 0.36-5.20 wt% In. The formulas of the zeolite products are expressed in Table 2 as a ratio of oxides per mole of $In_2O_3$. It will be noted that the $SiO_2/Al_2O_3$ mole ratio of each product always exceeded 1000.

8

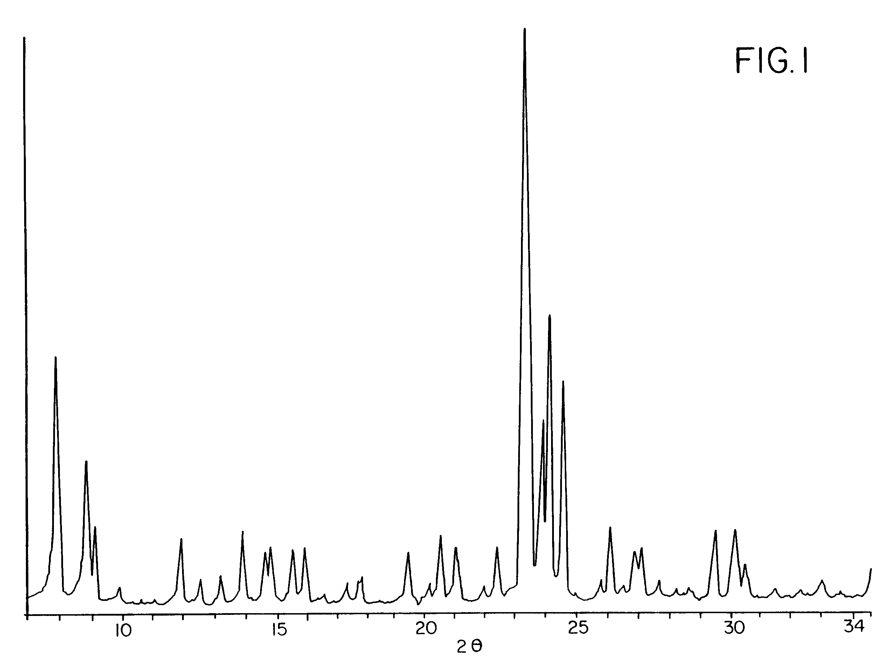

Figure 1 is the X-ray diffraction pattern for the In/ZSM-5 sample of Run No. 6.

## TABLE 2

### ANALYSES OF SOME INDIUM-CONTAINING ZEOLITIC SILICATE PRODUCTS

| Sample Run No. | Weight Percent | | | | | | | Moles C / Moles N | Moles per Mole $In_2O_3$ | | | |
|---|---|---|---|---|---|---|---|---|---|---|---|---|
| | C | N | Na | In | $SiO_2$ | $Al_2O_3$ | Ash | | $N_2O$ | $Na_2O$ | $Al_2O_3$ | $SiO_2$ |
| 12 | 6.96 | 0.66 | 3.28 | 5.20 | 62.47 | 0.070 | 85.34 | 12.3 | 1.04 | 3.15 | 0.03 | 46 |
| 11 | 6.74 | 0.43 | 2.64 | 4.19 | 69.94 | 0.24 | 86.20 | 18.3 | 0.84 | 3.14 | 0.13 | 64 |
| 13 | 7.02 | 0.56 | 0.79 | 3.48 | 76.45 | 0.035 | 84.78 | 14.6 | 1.32 | 1.13 | 0.02 | 84 |
| 7 | 8.02 | 0.71 | 0.98 | 2.11 | 74.85 | 0.078 | 88.05 | 13.6 | 2.36 | 2.29 | 0.06 | 132 |
| 6 | 8.01 | 0.68 | 1.48 | 2.14 | 74.64 | 0.11 | 88.72 | 13.7 | 2.61 | 3.45 | 0.11 | 133 |
| 10 | 7.93 | 0.74 | 0.56 | 2.26 | 83.85 | 0.005 | 88.05 | 12.4 | 2.68 | 1.23 | 0.009 | 142 |
| 8 | 8.37 | 0.81 | 1.83 | 1.92 | 73.14 | 0.025 | 88.36 | 12.0 | 3.46 | 4.76 | 0.03 | 146 |
| 9 | 8.22 | 0.62 | 0.54 | 1.49 | 82.14 | 0.031 | 85.96 | 15.5 | 3.41 | 1.81 | 0.05 | 211 |
| 2 | 8.12 | 0.69 | 0.40 | 0.36 | 78.05 | 0.083 | 85.69 | 13.7 | 15.7 | 5.55 | 0.52 | 830 |

### EXAMPLE 2

The In/ZSM-5 of Run No. 10 was prepared as follows:

A commercial silica gel (SPEX Ind.) with very low aluminum contamination was employed in the synthesis of In-ZSM-5. First, 0.85 g $In(NO_3)_3$ and 2.66 g NaOH pellets were dissolved in 180.2 g de-ionized water, then 5.64 g tetrapropylammonium bromide (TPABr) was dissolved in this basic solution. The solution was transferred to a 300 ml stainless steel autoclave, and 15.0 g of silica gel (SPEX) was added. The autoclave was then sealed and stirring and heating were begun. The hydrogel formed by this reaction mixture is described by the following mole ratios:

| | |
|---|---|
| $SiO_2/In_2O_3$ | 150 |
| $H_2O/SiO_2$ | 48 |
| $OH^-/SiO_2$ | 0.26 |
| $Na^+/SiO_2$ | 0.31 |
| $TPA^+/SiO_2$ | 0.10 |

The hydrogel was reacted at 160°C for 2 days at a stirring rate of 400 rpm before quenching. The resultant crystalline product was filtered, washed, and dried. X-ray powder diffraction analysis showed the product to be 100% crystalline ZSM-5, when compared to the diffraction pattern of a conventional ZSM-5. Elemental analysis of the ZSM-5 product gave: C = 7.93%, N = 0.74%, Na = 0.56%, In = 2.26%, Al = 0.005%, $SiO_2$ = 83.85%, Ash = 88.05%, all by weight.

These results expressed in mole ratios were: C/N = 12.5; moles/mole $In_2O_3$: $N_2O$ = 2.68, $Na_2O$ = 1.23, $Al_2O_3$ = .009, $SiO_2$ = 142.

Platinum incorporation was undertaken as follows: The as-synthesized zeolite was heated in nitrogen to 520°C at 1C/min and held there for 6 hours. It was then calcined in air in a similar manner. The calcined zeolite analyzed for 41.05% Si, 2.21% In (Si/In2 = 152), and 120 ppm Al, and sorbed 10.4% n-hexane at 90°C.

The calcined zeolite (3 g) was stirred in a solution of 150 mg $Pt(NH_3)_4Cl_2$ in 100 ml water at room temperature overnight. After being washed, filtered and dried, the ion-exchanged zeolite was found to contain 0.41 meq $NH_3$/g ash, which is equivalent to 1.89% Pt on sample. The platinum tetramine zeolite was then calcined in oxygen to 350°C at 0.5C/min and held there for 1 hour. Elemental analysis indicated the presence of 1.85% Pt on the final catalyst.

At very high hexane conversions (99%), benzene was formed in over 94% yield. Similarly, n-heptane yielded 96% toluene. Consistent with the non-acidic nature of this platinum catalyst, n-octane yielded predominantly ethylbenzene and ortho-xylene, 2-methylheptane produced mostly meta-xylene, and 3-methylheptane formed mainly ethylbenzene, para-, and ortho-xylene.

### EXAMPLE 3

Borosilicate ZSM-5 was synthesized at 170°C from a mixture of 12.4 g high purity silica (SPEX), 105 g 20% TEA hydroxide, and 0.8 g boric acid. The as-synthesized zeolite was then calcined in nitrogen and then in air at 520°C. The calcined zeolite contained 41.39% Si, 0.015% Al, and 0.44% B.

Two grams of the calcined borosilicate was impregnated with 135 mg $In(NO_3)_3$, and calcined in air at 500°C for 2 hours. 1.8 g of this material was then ion-exchanged with 28 mg $Pt(NH_3)_4Cl_2$ in 100 ml water at room temperature. TGA analysis in hydrogen indicated the presence of 0.18 meq N/g equivalent to 0.87% Pt. The platinum-exchanged zeolite was then calcined in oxygen to 350°C at 0.5°C/min.

The catalyst activity of the foregoing composition was examined. The "non-acidic" nature of the catalyst was confirmed by its ability to aromatize n-heptane to toluene in high yield. At 500°C and 30 torr (4 kPa) heptane in nitrogen, toluene was formed in 95% yield. Furthermore, the small amounts of both methane and propane produced were exceeded by the ethane formed, indicative of the low hydrogenolysis and acid activity of the catalyst.

| % Conversion | % Cl | % C2 % | Benzene | % Toluene(Selectivity) |
|---|---|---|---|---|
| 96 | 0.4 | 0.6 | 1.3 | 92 (96%) |
| 99 | 0.5 | 1.0 | 1.5 | 95 (96%) |

## EXAMPLE 4

Platinum incorporation into an indium-containing silicate of ZSM-5 structure was carried out by direct addition of a platinum compound to the zeolite synthesis reaction mixture as follows:

A solution was prepared by dissolving 2.00 grams of indium nitrate and 13.07 grams of NaOH pellets in 710.28 grams of de-ionized water. After the solids dissolved, 26.6 grams of tetrapropylammonium bromide (TPABr) was dissolved in the solution. Finally 1.29 grams of platinum tetraaminenitrate $[Pt(NH_3)_4(NO_3)_2]$ was dissolved in the solution, and the solution was transferred to a one-liter stainless-steel autoclave. Before sealing the autoclave, 66.67 grams of commercial silica gel (SPEX Industries) was poured into the autoclave. The autoclave was then sealed and heating and stirring were begun immediately. The reaction mixture hydrogel can be described by the following mole ratios:

| | |
|---|---|
| $SiO_2/In_2O_3$ | 300 |
| $H_2O/SiO_2$ | 40 |
| $OH^-/SiO_2$ | 0.30 |
| $Na^+/SiO_2$ | 0.33 |
| $TPA^+/SiO_2$ | 0.10 |
| $SiO_2/Pt$ | 300 |

The crystallization was carried out at 170°C with stirring (400 rpm).

After 4 days the autoclave was quenched in a water and ice bath to terminate the crystallization. The solid product was filtered, boiled in water, and finally filtered again before drying under a heat lamp. XRD analysis of the solid product showed the material to be crystalline zeolite ZSM-5.

Chemical analysis of the indium-containing ZSM-5 product gave:

| Weight Percent | | | | | | | |
|---|---|---|---|---|---|---|---|
| C | N | Na | In | Pt | $SiO_2$ | $Al_2O_3$ | Ash |
| 8.27 | 0.74 | 1.3 | 1.1 | 0.52 | 82.7 | 0.0265 | 85.05 |

which gave:

| Moles C | Moles per Mole $In_2O_3$ | | | | |
|---|---|---|---|---|---|
| Moles N | $N_2O$ | $Na_2O$ | $Al_2O_3$ | $SiO_2$ | Pt |
| 13.1 | 5.52 | 5.90 | 0.05 | 288 | 0.55 |

## EXAMPLE 5

The synthesis of a binary oxide zeolite having the structure of ZSM-5 was carried out in the two-phase system. The aqueous phase of the two-phase system comprised 2.8 g $In(NO_3)_3xH_2O$ dissolved in 35 g water to which was added 63 g TPAOH (40% in $H_2O$). Constituting the organic phase was 77.0 g $Si(OCH_3)_4$ dissolved in 35 g of 1-hexanol. The mixture was nucleated at 180°C for 24 hours and crystallized at 200°C for 144 hours. The final product was filtered and washed. The X-ray diffraction pattern of the dried material proved it to be well-crystallized ZSM-5.

The sample was ammonium-exchanged (1 M $NH_4Cl$, twice, 60°C, 20 ml/g zeolite) and calcined. The chemical composition of the ash of a 1000°C calcined sample was 79.3 wt. % $SiO_2$ and 1.5 wt. % $In_2O_3$. The ash residue also contained a small quantity, i.e. 85 ppm, of aluminum.

Temperature-programmed desorption of ammonia indicated an exchange capacity of 0.09 meq/g for the product of this example. The Si/In ratio from TPAD was 190.5. The sample had an Alpha Value of 1.0.

## EXAMPLE 6

The synthesis of Example 5 was repeated, except that the mixture contained 3.6 g In(NO$_3$)$_3$.xH$_2$O in the aqueous phase. The product material was filtered and dried. It had the same characteristic ZSM-5 X-ray lines as the product of Example 5. The material was calcined and ammonium-exchanged as described in Example 5. The chemical composition of the ash of a 1000°C calcined sample was 78.2 wt. % SiO$_2$ and 3.1 wt. % In$_2$O$_3$. The ash residue also contained a small quantity, i.e. 180 ppm, of aluminum.

Temperature-programmed desorption of ammonia indicated an exchange capacity of 0.21 meq/g for the product of this example. The Si/In ratio from TPAD was 77.9. The sample had an Alpha Value of 2.5.

## EXAMPLES 7-11

The synthesis of Example 5 was repeated, except that the mixtures contained varying amounts of In-(NO$_3$)$_3$.xH$_2$O. Five preparations were made, with the following compositions:

| Example | 7 | 8 | 9 | 10 | 11 |
|---|---|---|---|---|---|
| **Aqueous Phase(g)** | | | | | |
| H$_2$O | 40.0 | 40.0 | 35.0 | 40.0 | 40.0 |
| In(NO$_3$)$_3$x3H$_2$O | 0.9 | 7.2 | 1.8 | 1.8 | 3.6 |
| TPAOH, 40% | 63.0 | 63.0 | 63.0 | 63.0 | 63.0 |
| **Organic Phase(g)** | | | | | |
| 1-Hexanol | 60.0 | 60.0 | 35.0 | 60.0 | 60.0 |
| Si(OCH$_3$)$_4$ | 77.0 | 77.0 | 77.0 | 77.0 | 77.0 |

The product materials were filtered and dried. They had the same characteristic X-ray lines as ZSM-5. The materials were calcined and ammonium-exchanged as in Example 5. Their properties were as follows:

| Example | 7 | 8 | 9 | 10 | 11 |
|---|---|---|---|---|---|
| SiO$_2$, wt. % | 84.0 | 77.5 | 80.5 | 76.7 | 82.5 |
| In$_2$O$_3$, wt. % | 0.67 | 5.1 | 1.58 | 1.31 | 2.92 |
| Al, ppm | 105 | 65 | 130 | 85 | 60 |
| Exchange Capacity, meq/g | 0.09 | 0.17 | 0.17 | 0.12 | 0.21 |
| Si/In (from TPAD) | 193 | 99 | 95 | 138 | 77 |
| Alpha Value | 1.5 | 1.6 | 1.0 | 1.0 | n.d. |

## EXAMPLE 12

A hydrotreated Arab straight-run (LSR) naphtha was reformed over an indium containing ZSM-5 catalyst comprising 2.3 wt.% platinum, 2.88 wt.% indium, 0.45 wt.% sodium and less than 360 ppmw aluminum, with the remainder being silica. The Arab light LSR naphtha used was a C$_6$ - 210°F (99°C) fraction, containing 42.5 wt.% C$_6$ paraffins, 32.9 wt.% C$_7$ paraffins, and an RON+O of 51 (calculated). Additional properties and compositional details are described in the following table:

## FEED PROPERTIES
### ARAB LIGHT LSR NAPHTHA

| | |
|---|---|
| API Gravity | 73.5 |
| Sulfur, ppmw | 0.06 |
| Nitrogen, ppmw | 0.2 |
| Octane RON+O | 51 |
| Distillation, D-86 | |
| 5% vol., °F(°C) | 157 (69) |
| 50% vol., °F(°C) | 171 (77) |
| 95% vol., °F(°C) | 203 (95) |
| Composition, %wt. | |
| $C_5$ paraffins | 3.3 |
| $C_6$ parffins | 41.4 |
| $C_6$ naphthenes | 7.4 |
| $C_6$ aromatic | 2.1 |
| $C_7$ paraffins | 32.3 |
| $C_7$ naphthenes | 7.1 |
| $C_7$ aromatic | 3.1 |
| $C_8$ + PNA | 3.3 |

Reforming of the Arab Light LSR naphtha over the platinum-indium ZSM-5 catalyst was conducted at 540°C (1000°F), 446 kPa (50 psig), 1.0 LHSV, and 5:1 $H_2$/HC mole ratio at the reactor inlet. Under these conditions reforming resulted in 84.9% wt. yield of $C_5$ + gasoline at 93.6 RON+O. The $C_5$ + gasoline contained 56.8% wt. aromatics.

**EXAMPLE 13**

Under the same reaction conditions as in Example 12, but in the absence of added hydrogen, the RON+O of the $C_5$ + gasoline increased to 101.8, while the yield remained nearly constant at 84.18%wt. The aromatic content of the $C_5$ + gasoline increased to 72.9% wt. It should be noted that no change in performance of the ZSM-5 catalyst was observed in the absence of added hydrogen from 137 to 192 hours on stream at 540°C (1000°F), 446 kPa (50 psig) and 1.0 LHSV while achieving an average 101 RON+O $C_5$ + gasoline in 83.5% wt. yield. Data concerning catalyst stability are set forth below.

| Catalyst Stability | | |
|---|---|---|
| Time on Stream, hours | 136.8 | 192.0 |
| $C_5$ + Gasoline Yield, %wt. | 83.1 | 84.1 |
| $C_5$ + Gasoline Yield, %vol. | 70.6 | 71.6 |
| Octane, RON+O | 100.0 | 101.8 |
| $H_2$ produced, SCF/B ($Nm^3/n^3$) | 1980 (352) | 1990 (354) |

The results of Examples 12 and 13 constitute significant yield and octane advantages when compared with the results expected for processing the LSR naphtha in a conventional semi-regenerative reformer, using a chlorided platinum/alumina catalyst. Typically, conventional reforming of this naphtha would result in 61% wt. yield of 93 RON+O $C_5$ gasoline at reaction conditions of 540°C (1000°F), 1724 kPa (250 psig), 1.0 LHSV and 10:1 $H_2$/HC mole ratio, with the $C_5$ + gasoline product containing only 51.2% wt. aromatics. Additional details for comparison are set forth in Table 3 below.

13

## TABLE 3

| Catalyst | Pt-Cl/alumina | Pt/In ZSM-5 | |
|---|---|---|---|
| Process Conditions | | | |
| Temperature, °F(°C) | 1000( 540) | 1000(540) | 1000(540) |
| Pressure, psig (kPa) | 250(1724) | 50(4460 | 50(446) |
| LHSV, 1/hr | 1.0 | 1.0 | 1.0 |
| $H_2$/HC mole ratio | 10 | 5 | --- |
| Process Yields, wt% | | | |
| Hydrogen | 0.6 | 3.3 | 4.6 |
| $C_1$-$C_4$ | 38.3 | 11.8 | 11.3 |
| $C_5$+ Gasoline | 61.1 | 84.9 | 84.1 |
| $C_5$+ Product Quality | | | |
| Octane, RON+O | 93.0 | 93.6 | 101.8 |
| Aromatics, wt% | 51.2 | 56.8 | 72.9 |

Reducing the reaction pressure and hydrogen circulation would also improve the selectivity of the conventional reforming catlyst, but would result in unacceptably high aging rates. The ability of the platinum containing indium ZSM-5 catalyst to operate in the absence of added hydrogen appears to offer significant advantes, when compared not only to semi-regenerative, but also cyclic reforming processes using conventional catalysts.

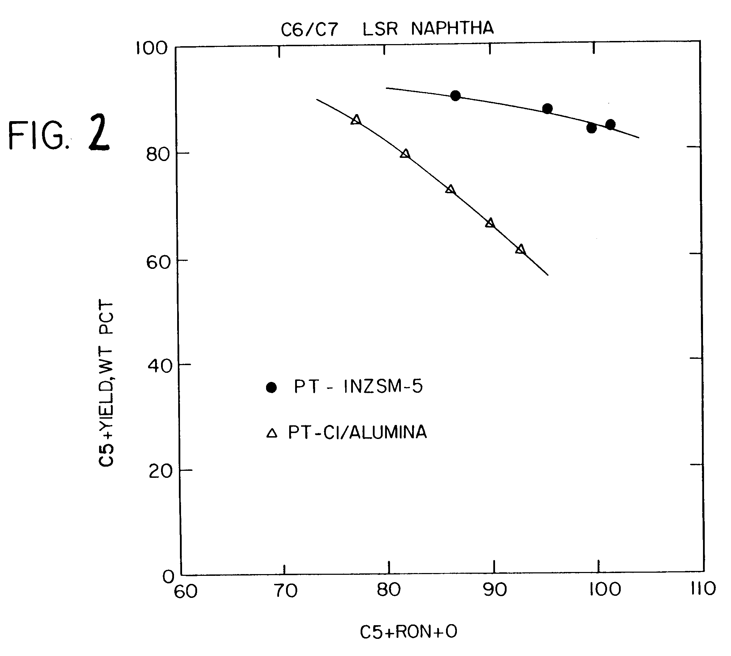

Figure 2 illustrates the yield vs RON curve obtained using the reforming process of example 13 as compared with the conventional chlorided Pt/Al$_2$O$_3$ catalyst.

## EXAMPLE 14

Example 12 was repeated but with hydrogen partial pressure in the reactor being reduced by the addition of a helium diluent. The data given below compares the results of examples 12 - 14.

| | Example 12 | Example 13 | Example 14 |
|---|---|---|---|
| Inlet Gas/HC Feed | | | |
| mole ratio | 5:1 $H_2$/HC | NO ADDED $H_2$ | 5:1 He/HC |
| Hydrogen, %wt. | 3.3 | 4.6 | 5.7 |
| C1-$C_4$ | 11.8 | 11.3 | 3.8 |
| $C_5$+ Gasoline | 84.9 | 84.1 | 90.5 |
| $C_5$+ RON+O | 93.6 | 101.8 | 103.3 |
| Aromatics, %wt. | 48.0 | 61.0 | 68.7 |

It will be seen that introducing an inert carrier, in this example helium, to reduce the partial pressure of hydrogen in the reactor results in additional yield and octane gains by further improving aromatics selectivity. Hydrogenolysis and cracking reactions which result in $C_1$-$C_4$ make are suppressed. Both hydrogen yield and purity increase as a result.

## EXAMPLE 15

In this example, the pretreated light paraffinic naphtha used in the preceding Example was processed over a silica-bound Pt/In ZSM-5 catalyst while cofeeding hydrogen and propane. A second experiment using hydrogen only as a cofeed with the naphtha is shown for comparison. Reaction conditions were 540°C (1000°F), 446 kPa (50 psig) and 1.0 LHSV on naphtha feed, with 12:1 hydrogen or hydrogen + propane to naphtha mole ratio at the reactor inlet. The naphtha partial pressure (4 psi or 28 kPa) was therefore the same in both experiments, while the hydrogen partial pressure was lowered from 317 kPa (46 psi) to 83 kPa (12 psi) by cofeeding propane as the diluent.

| LOW PRESSURE REFORMING WITH PROPANE COFEED | | | |
|---|---|---|---|
| | Feed | $H_2$ Only | $H_2$ + $C_3$ |
| $H_2/C_5$ + HC Mole Ratio | -- | 12:1 | 3:1 |
| $C_3/C_5$ + HC Mole Ratio | -- | -- | 9:1 |
| $C_3$ vol. % on naphtha | 577.1 | 11.1 | 546.0 |
| $C_3$ = Yield, vol. % | -- | 0.7 | 33.0 |
| $C_5$ + Gasoline Yield, vol. % | 100.0 | 75.3 | 81.0 |
| $C_5$ + Gasoline Octane RON+O | 51 | 83 | 91 |
| Hydrogen Produced, SCF/B ($Nn^3/m^3$) | -- | 580(103) | 1954(348) |
| Hydrogen Purity | -- | 79.0 | 96.9 |
| Aromatics, wt.% | 5.2 | 29.4 | 44.9 |

Cofeeding propane to reduce the hydrogen partial pressure improves aromatization selectivity and results in both increased gasoline yield and higher (calculated) octane. Dehydrogenation of the propane cofeed to propylene and hydrogen approaches thermodynamic equilibrium at the reaction conditions chosen. There is significant increase in the hydrogen purity (defined as moles hydrogen relative to total moles hydrogen, methane and ethane), and the quantity of hydrogen produced. Note that the propane diluent and higher hydrocarbons are easily separated from the hydrogen produced, thereby ensuring economical recovery of the high purity hydrogen.

The catalyst used in this experiment was prepared by extruding an as-synthesized TPANa (In)ZSM-5 50/50 with silica according to the method of U.S. 4,582,815. The extrudate was calcined in nitrogen and then air at 538°C and then ion-exchanged with platinum tetrammine chloride solution, which was then calcined in oxygen from 25 to 350°C at 0.5°C/min and held at 350°C for 1 hour. the calcined catalyst which resulted contained 0.48 wt.% platinum, 0.49 wt.% indium, 0.11 wt.% aluminum, and 0.12 wt.% sodium.

## EXAMPLE 16

Post-processing of a reformate of 70 RON+O over a platinum indium silicate having an X-ray diffraction pattern of ZSM-5 was undertaken.

The reformate used as feed was obtained by sampling the second reactor of a three reactor reforming process in which the reforming catalyst was a conventional chlorided platinum catalyst. The paraffins which would have been converted in the third reactor remained, resulting in the relatively low octane number. This reformate was characterized by a research octane number of 67.7, a combined hydrogen content of 13.75%, and contained 2.7 wt.% $C_5$ paraffins, 52.4 wt.% $C_6$ + paraffins, 10.6 wt.% naphthenes, and 33.7 wt.% aromatics.

The platinum containing indium ZSM-5 catalyst used was that employed in Example 12 after approximately 18 days on stream.

The following comparison shows the results observed when post-processing low octane reformate at 540°C (1000°F), 446 kPa (50 psig) and 1.0 LHSV, with either hydrogen or helium at the inlet.

| POST-PROCESSING REFORMATE | | | |
|---|---|---|---|
| | Feed | 5:1 $H_2$/HC | 5:1 He/Hc |
| Hydrogen, wt.% | -- | - 0.2 | 1.6 |
| $C_1$-$C_4$ | -- | 15.5 | 1.9 |
| $C_5$ + Gasoline | 100.0 | 84.5 | 96.5 |
| $C_5$ + RON + O | 67.7 | 89.0 | 93.2 |
| $C_5$ + Aromatics, wt.% | 34.6 | 43.9 | 57.4 |

From the results above, it is noted that post-processing of the low octane paraffinic reformate over the platinum containing indium ZSM-5 catalyst clearly improves the gasoline octane. Reducing the hydrogen partial pressure in the reactor by cofeeding a diluent stream improves the aromatics selectivity, and results in both higher gasoline yield and octane.

## EXAMPLE 17

The Pt/In-ZSM-5 of Example 2 was used to catalyze the conversion of n-octane in a nitrogen diluent to aromatics including styrene. Higher temperatures and great dilution led to improved sytrene yields as shown below.

| Octane Pressure | Temp °C | Conversion | Styrene Yield |
|---|---|---|---|
| 10 torr (1.3 kPa) | 450 | 99.3% | 4.8% |
| 10 torr (1.3 kPa) | 500 | 95.4% | 17.9% |
| 10 torr (1.3 kPa) | 538 | 98.4% | 33.2% |
| 1 torr (133 kPa) | 550 | 99.4% | 64.3% |

## EXAMPLE 18

A further sample of the Pt/In ZSM-5 catalyst employed in Example 12 (40.42% Si, 2.88% In, 0.45% Na, 358 ppm Al, and 2.3% Pt) was used to dewax a heavy neutral raffinate feed having a pour point of 49°C (120°F). The properties of the feed are listed in Table 4.

## TABLE 4

Charge Stock

| H-NMR | PCT | 14.3 |
|---|---|---|
| Nitrogen-Chemiluminesce | | 7 PPM |
| Basic Nitrogen-TITN | | 3 PPM |
| Sulfur by XRF, 0.002-5% | PCT | 0.02 |
| API Gravity | | 31.5 |
| Refractive Index Liquids | | 1.458 |
| Flash PT Cleve Open Cup | | 505 |

| Kinematic Viscosity (100°C) | 9.648 |
|---|---|
| Kinematic Viscosity (300°F) (149°C) | 3.991 |

Arom by Silica Gel

| Percent Recovered | 83.98 |
|---|---|
| Percent Residue | 15.44 |
| Percent Loss | 0.58 |
| Percent Non-Aromatics | 84.47 |
| Percent Aromatics | 15.53 |

Vacuum Dist.

| Initial Boiling Point | 714°F (379°C) |
|---|---|
| 5 Vol. Percent Distilled | 853°F (456°C) |
| 10 Vol. Percent Distilled | 878°F (470°C) |
| 20 Vol. Percent Distilled | 890°F (477°C) |
| 30 Vol. Percent Distilled | 905°F (485°C) |
| 40 Vol. Percent Distilled | 921°F (494°C) |
| 50 Vol. Percent Distilled | 936°F (502°C) |
| 60 Vol. Percent Distilled | 954°F (512°C) |
| 70 Vol. Percent Distilled | 979°F (526°C) |
| 80 Vol. Percent Distilled | 1010°F (543°C) |
| 90 Vol. Percent Distilled | 1053°F (567°C) |
| 95 Vol. Percent Distilled | 1086°F (586°C) |
| Percent Recovered | 98.0 |
| End Point | 1126°F (608°C) |

Dewaxing was carried out in a continuous flow microreactor at about 538°C, 1480 kPa (200 psig) and 0.5 WHSV. The $H_2$:oil ratio was 1.9. The product yields are shown in Table 5 below.

17

TABLE 5

| Product Yields from Dewaxing | |
|---|---|
| Days on Stream | 4.8 |
| Lube Yield (650°F) (343°C) | 62.5% |
| Kerosene Yield (330-650°F) (166-343°C) | 14.5% |
| Naphtha Yield (125-330°F) (52-166°C) | 19.0% |
| Total Liquid Yield | 96.0% |

A chromatogram of the lube and kerosene fraction showed that the waxy paraffins had been converted from the 343° + C (650° + F) range into the distillate range. The pour point of the 343° + C (650° + F) lube material was -6°C (22°F), as compared to the feed pour point of 49°C (120°F). Furthermore, shifting the paraffinic material into the distillate range produced a high quality distillate with an estimated cetane number of 55.

**EXAMPLE 19**

Tin ZSM-5 silicate was synthesized in a static system at 149°C (300°F). 400 g 28.5% sodium silicate (Q-brand) was added to a solution of 60 g 50% tetramethylammonium choride, 15 g $SnCl_4/5H_2O$, 30 g 98% $H_2SO_4$, and 60 g tetrapropylammonium bromide in 2250 g water. The mixture was stirred and then placed in a polypropylene bottle in an autoclave for 5 days. The product was 85% crystalline ZSM-5 and consisted of large 5 - 10 micron crystals. In this and following preparations the zeolitic silicates produced were characterized as having at least one crystal dimension which was at least 0.5 microns; it analyzed for 80.4% $SiO_2$, 0.30% $Al_2O_3$, 3.78% Sn, 2.00% Na, 7.70% C, and 1.05% N.

**EXAMPLE 20**

Another tin containing ZSM-5 sample was synthesized by dissolving 0.69 g Sn(II)$SO_4$ in 170 g de-ionized water and then adding 3.39 g NaOH. To this was added 6.38 g tetrapropylammonium bromide. The mixture was transferred to a 300 ml stainless steel autoclave and 16.0 g of a low aluminum content silica gel (SPEX Ind.) was added with stirring. The hydrogel formed by this reaction mixture is described by the following mole ratios:

| $SiO_2/Sn$ | $H_2O/Sn$ | $OH-SiO_2$ | $Na+/SiO_2$ | $TPA+/SiO_2$ |
|---|---|---|---|---|
| 75 | 40 | 0.30 | 0.35 | 0.10 |

The hydrogel was reacted at 160°C for 5 days with stirring (400 rpm) before quenching. The resulting crystalline product was processed in the usual manner by filtering, washing, and drying. X-ray diffraction analysis of the product zeolite showed it to be 100% crystalline ZSM-5. SEM indicated an average crystal size greater than 2 microns.

**EXAMPLE 21**

A tin containing ZSM-5 sample was synthesized in a similar manner to Example 20 except that the $SiO_2/Sn$ ratio was 150 and the $Na+/SiO_2$ was 0.31. The crystalline ZSM-5 product cotained 1.36% Sn, 0.0025% Al, 0.93% Na, and 89.31% Ash.

**EXAMPLE 22**

A tin containing ZSM-5 sample was synthesized in a similar manner to example 20 except that the $SiO_2/Sn$ ratio was 50, the $Na+/SiO_2$ was 0.38, and the synthesis time was 4 days.

18

## EXAMPLE 23

A tin containing ZSM-5 was synthesized at a $SiO_2/Sn$ ratio of 38, a $Na+/SiO_2$ ratio of 0.40, and a synthesis time of 3 days.

Tin incorporation was achieved during zeolite synthesis in Examples 19 - 23; i.e., tin salts were added directly to the high silica ZSM-5 synthesis mixture. SEM data suggests that a significant portion of the tin is located outside of the large crystals formed. Nevertheless, some tin must be inside the ZSM-5 crystals, since it modifies the selectivity of the platinum, which itself is intracrystalline.

## EXAMPLE 24

Platinum incorporation into the silicates of Examples 19 - 23 was undertaken. The as-synthesized tin silicates were calcined first in nitrogen and then in air at 520°C. The calcined materials were ion-exchanged with aqueous $Pt(NH_3)_4Cl_2$ at room temperature; typically, 15 - 20 mg per gram silicate was used in a non-acidic aqueous medium. The platinum tetramine-containing silicates were then calcined in oxygen to 350°C at 0.5 C/min.

Elemental analysis of the tin silicate of Example 20 after platinum incorporation indicated Pt = 0.92%, Sn = 2.7%, Na = 0.89%.

Elemental analysis of the tin silicate of Example 19 after platinum incorporation, Pt = 0.65%, Sn = 3.50%, Al = 0.093%.

Elemental analysis of the tin silicate of Example 21 after platinum incorporation indicated Pt = 0.80%, Sn = 1.54%, Al = 31ppm.

## EXAMPLE 25

A solution of 11.3 g $SnCl_2 \cdot H_2O$ in 100 ml methanol was formed. To that solution was added 20 g of a sample of H-ZSM-5 (silica:alumina ratio of 70:1). Then 2.5 ml of an aqueous solution of $H_2PtCl_6$ (1.3g) was also added to the solution. The mixture was allowed to stand for 4 hours. The product was decanted, washed with 10 x 100 ml 3A denatured alcohol and let stand overnight under 100 ml 3-A. [3-A refers to a dessicant by Linde]. The product was decanted, washed with 100 ml 3-A and dried in an oven at 100°C.

The composition of Example 25 was acidic and thus not a part of the invention. The composition of Example 25 exhibited different properties from the non-acid platinum tin ZSM-5 of the invention as shown below.

| | Non-Acidic Catalyst of the Invention | Acidic Composition of Ex. 25 |
|---|---|---|
| Alpha Value in He 1 hr on stream | 424 | 62 |
| Benzene selectivity from alpha value test | 50.4 | 2.0 |

In the above composition, hexane was the feed and the numerical value, referred to as alpha in the foregoing table, is used as a measure of the hexane conversion activivy. From the foregoing results it can be noted that the composition in accordance with the invention exhibited greater hexane conversion activity than the Example 25 composition and in the alpha value test the catalyst of the invention exhibited greater selectivity for hexane conversion to benzene.

## EXAMPLE 26

The ability of some of the catalyst of Example 24 to aromatize n-heptane to toluene was assessed at 538°C and 30 torr (4 kPa) heptane in nitrogen. Heptane was introduced into the reactaor in a nitrogen stream passing through a vaporizer containing heptane at 15 - 20°C. The presence of tin in these Pt/ZSM-5 catlysts greatly increased the toluene yield and suppressed the amount of methane formed. Some of the data obtained is shown below; scandium, titanium, and boron-containing Pt/ZSM-5 catalysts, prepared in a similar manner, are included for comparison purposes. Yields shown are on a hydrogen-free weight basis.

19

| Catalyst | SnZSM-5 Source | Conversion | $CH_4$ Yield | Yield | Selectivity |
|---|---|---|---|---|---|
| Pt/SnZSM-5 | Ex.21 | 91.7% | 6.5% | 53.0% | 57.8% |
| Pt/SnZSM-5 | Ex.19 | 95.1% | 1.5% | 82.3% | 86.5% |
| Pt/SnZSM-5 | Ex.20 | 97.7% | 0.3% | 95.4% | 97.7% |
| Pt/SnZSM-5 | Ex.22 | 99.7% | 0.6% | 94.5% | 94.8% |
| Pt/SnZSM-5 | Ex.23 | 98.4% | 0.2% | 96.4% | 98.1% |
| Pt/ScZSM-5 | | 96.3% | 15.4% | 37.5% | 38.9% |
| Pt/TiZSM-5 | | 96.1% | 18.9% | 30.6% | 31.8% |
| Pt/B-ZSM-5 | | 94.7% | 19.6% | 28.6% | 30.2% |

## EXAMPLE 27

The platinum-exchanged catalysts of examples 20 and 23 were used to effect reforming of a hydrotreated Arab light naphtha, boiling range 82 - 121°C (180-250°F), at 540°C (1000°F) and atmospheric pressure (100 kPa) in nitrogen. The results are shown below.

| Catalyst | Pt/Sn-ZSM-5 | Pt/Sn-ZSM-5 |
|---|---|---|
| Sn/ZSM-5 Source | Ex. 20 | Ex. 23 |
| WHSV | 4.0 | 2.0 |
| $N_2$/HC | 3 | 4 |

| | Feed Composition | Product ($H_2$-free wt. basis) | |
|---|---|---|---|
| $C_1$-$C_4$ | 0 | 0.74% | 0.69% |
| 2-MeC$_5$ | 9.35% | 5.75% | 5.31% |
| 3-MeC$_5$ | 7.11% | 4.32% | 3.88% |
| n-C$_6$ | 24.22% | 3.27% | 5.13% |
| BENZENE | 2.12% | 24.35% | 24.83% |
| 2-MeC$_6$ | 8.41% | 5.24% | 4.69% |
| 3-MeC$_6$ | 7.22% | 3.90% | 3.52% |
| n-C$_7$ | 17.05% | 1.21% | 3.17% |
| TOLUENE | 3.23% | 21.62% | 24.21% |
| Selective conversion of the normal paraffins to aromatics was observed. | | | |

## EXAMPLE 28

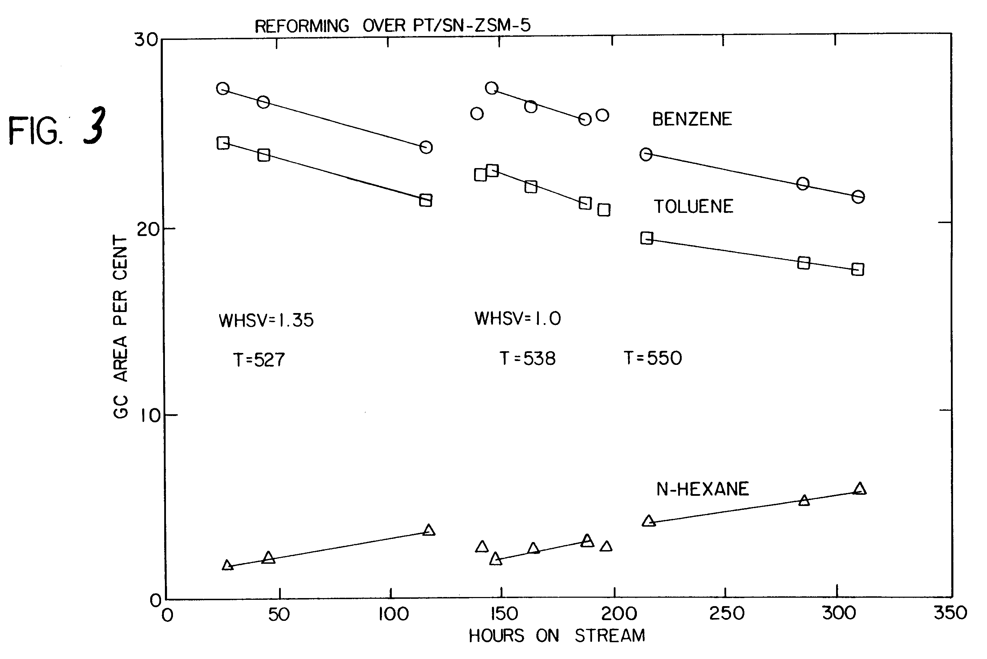

An extended light naphtha reforming run was conducted over a 0.9% Pt/Sn-ZSM-5 catalyst of Example 20 as shown in Figure 3. The initial inlet temperature was 527°C, and this was increased incrementally to 550°C. WHSV was 1.35 initally, and later, 1.0. The on-line GC yields of both benzene and toluene are shown in Figure 3. Formation of $C_1$-$C_4$ light gases was quite low; at 45 hours on stream light gas formed was about 0.6%, and about 1.5% at 300 hours on stream.

Throughout the run, the liquid product was collected in a $CaCl_2$-ice bath at approximately -40°C. Overall liquid recovery was 90 - 92 weight per cent of feed. The measured research octane ratings (RON + O) of the various fractions collected ranged from 97 after day 1 on stream to better than 92 after 12 days. The liquid products contained significant amounts of olefins as indicated by their bromine numbers which ranged from 44 to 33.

The very high aromatization selectives of these platinum/tin ZSM-5 catalysts, and their apparent stability in the absence of added hydrogen, make the ideal candidates for reforming catalysts.

## EXAMPLE 29

The aromatization of a hydrotreated $C_6$ - $C_7$ light naphtha was investigated over a 1.6% Pt/Sn-ZSM-5 (analysis of which indicated 1.6% Pt; 3.0 Sn; 0.64 Na and less than 59 ppm $Al_2O_3$) in hydrogen at a $H_2$/HC ratio of 1 at atmospheric pressure and 1 WHSV. The temperature ranged from 520 - 538°C over a period of 14 days. Liquid products were recovered in better than 90 wt.% yield based on feed. The RON's of the collected products were 97 - 98.

The above run was continued at 239 kPa (20 psig) and 538 - 550°C. The liquid product recovered in 85 wt.% yield after a total of 25 days on stream exhibited a RON of 97 and MON (motor octane number) of 83.

## EXAMPLE 30

A silica-bound extrudate Pt/Sn-ZSM-5 was prepared and used for naphtha reforming. A high silica tin-containing ZSM-5 was synthesized as described earlier. It contained 6.84% C, 0.61% N, 5.31% Sn, 0.0057% Al, 1.04% Na, and 79.9% $SiO_2$. The silica-bound extrudate, containing 35% silica binder, was prepared according to U.S. Patent No. 4,852,815.

The dry extrudate was calcined in nitrogen at 540°C (1000°F) and then ion-exchanged with $Pt(NH_3)_4Cl_2$. The platinum-containing catalyst was then calcined in $O_2$ at 350°C. The final catalyst composition analyzed for 0.78% Pt, 3.66% Sn, 0.33% Na, and 0.23% $Al_2O_3$.

A pretreated $C_6$/$C_7$ light naphtha was reformed over the above catalyst in hydrogen at a $H_2$/HC ratio of 1, at least 1 WHSV, atmospheric pressure, and 538°C. The liquid product recovered in better than 90 wt.% yield has a RON clear of 95.6.

## EXAMPLE 31

A mixture of $C_{6-C9}$ normal paraffins (25% $C_6$, 29% $C_7$, 25% $C_8$, and 20% $C_9$) was reformed over a Pt/Sn-ZSM-5 (analysis of which indicated 1.5% Pt; 2.7% Sn; 0.63% Na and 72 ppm $Al_2O_3$) catalyst in hydrogen at a $H_2$/HC ratio of 1, at atmospheric pressure, 1 WHSV, and 538°C. The liquid product which was recovered in 84 wt.% yield had a RON of 100. The Sn-ZSM-5 was prepared according to Example 20.

## EXAMPLE 32

A model feed consisting of a 3:1 wt. ratio of n-heptane and methylcyclopentane was reformend over a non-acidic Pt/Sn-ZSM-5 catalyst containing 1.5% Pt, 2.7% Sn, 0.63% Na, and 72 ppm $Al_2O_3$. Conditions were 1 WHSV, 860 kPa (110 psig) and 538°C. The diluent ratios used were 4:1:1 and 6:1:1 $N_2$:$H_2$:HC. It was found that when the diluent ratio was increased, the yield of aromatics (toluene) increased while the amount of residual unreacted heptane decreased. At the same time, loss to $C_1$-$C_5$ light hydrocarbons decreased from 6.5 wt.% to 4.0 wt.%

## EXAMPLE 33

The Pt/Sn-ZSM-5 of Example 30 was used to effect dehydrogenation of n-pentane using a down-flow glass reactor containing 1.2 g of the catlyst. Pentane was introduced into the reactor in a nitrogen stream passing through a vaporizer containing pentane at 0°C. The reaction was conducted at 538°C, 1 atmosphere (100 kPa) pressure, and 27 kPa (200 torr) pentane in nitrogen.

After about 20 hours on stream, the conversion of n-pentane was 68%, with loss to $C_4$- hydrocarbons less than 1.7%. Compositional analysis of the liquid collected over the period of 2.5 to 231 hours on stream is shown in Table 6 below.

TABLE 6

| Liquid Product Composition | |
|---|---|
| Component | Weight Percent |
| $C_1$ - $C_4$ | 1.5% |
| n-Pentane | 25.5% |
| iso-Pentane | 1.3% |
| Pentene-1 | 6.7% |
| trans-Pentene-2 | 16.6% |
| cis-Pentene-2 | 9.3% |
| 3-Methylbutene-1 | 0.9% |
| 2-Methylbutene-1 | 4.4% |
| 2-Methylbutene-2 | 8.0% |
| Cyclopentane | 0.4% |
| Cyclopentene | 1.5% |
| Cyclopentadiene | 7.9% |
| Non-cyclic Dienes | 16.0% |

The results suggest that dehydrogenation and dehydrocyclization compete effectively with hydrogenolysis over this catalyst, and that they also dominate over skeletal isomerization.

The measured clear RON of the recovered liquid product was 97.1. The actual RON of the dehydrogenation products of pentane must be even higher, since the total product still contained 25% n-pentane which has a RON of 63.

## EXAMPLE 34

A stream of ethylbenzene (approximately 10 torr (1.3 kPa)) in nitrogen was passed over a 1.8% Pt/Sn-ZSM-5 catalyst at atmospheric pressure, 0.4 WHSV, and 538°C. On-line GC analysis indicated a styrene yeild of 74% with a selectivity of 89%.

## EXAMPLE 35

An aged sample of the catalyst used in Example 34 was used to study the dehydrogenation of 3-methyl-pentane at atmospheric pressure, 1 WHSV, and 538°C in a mkixture of $N_2$ and $H_2$ at a ratio of 4:1:1 relative to hydrocarbon. At 20% conversion, selectivity to methylpentenes was about 65%.

## EXAMPLE 36

The catalyst of Example 35 was used to study the dehydrogenation of methylcyclopentane under similar conditions except that the total pressure was 790 kPa (1000 psig). At 18% conversion, methylcyclopentene was observed as the major product in about 45% selectivity.

The upgrading of n-pentane via dehydrogenation to olefinic and cyclic $C_5$ hydrocarbons over non-acidic tin-modified Pt/ASM-5 catalysts appears to offer an attractive alternative to pentane isomerization as a means of octane enhancement of this fraction, while at the same time reducing the total vapor pressure.

Whereas isomerization of n-pentane to an equilibrium mixture is isopentane and n-pentane yields a mixture having a clear RON of about 87, dehydrogenation and dehydrocyclization produced a liquid of 97 RON, despite containing 25% n-pentane. About 10% of the product formed over Pt/Sn-ZSM-5 consisted of cyclopentyl compounds, 33% linear olefins, 13% branched olefins, and about 16% non-cyclic dienes.

## EXAMPLE 37

Thallium ZSM-5 was synthesized by dissolving 0.85 g $TlNO_3$ in 170.6g deionized water and then adding 2.05 g NaOH pellets. After all the base had dissolved, 6.38 g tetrapropylammonium bromide (TAPBr) was added. The resulting solution was transferred to a 300 ml stainless steel autoclave and 16.0 g of silica gel (SPEX Ind.) was stirred into the solution. The hydrogel produced can be described by the following mole ratios:

22

| SiO$_2$/Tl$_2$0 | H$_2$O/SiO$_2$ | OH-/SiO$_2$ | TPA + /SiO$_2$ |
|---|---|---|---|
| 150 | 40 | 0.20 | 0.10 |

The hydrogel was heated in the autoclave for 4 days at 160°C, with stirring at 400 rpm. The product was filtered, washed, and dried. X-ray diffraction analysis indicated it to be 100% crystalline ZSM-5.

Elemental analysis indicated the presence of 8.26% C, 1.88% H, 0.74% N, 0.34% Na. 4.33% Tl, 80.65% SiO$_2$, and 0.00095 Al in the ZSM-5 prudct.

Catalyst preparation was undertaken as follows: The as-synthesized thallium silicate was calcined, first in nitrogen and then in air, at 520°C. The calcined zeolite containted 2.43% Tl, 38 ppm Al, and 43.15% Si.

Platinum was incorporated by ion exchange with Pt(NH$_3$)$_4$Cl$_2$ (15 mg/g zeolite) at room temperature. TGA ammonia titration in hydrogen indicated the presence of 0.67% Pt. The platinum-containing zeoliet was then hated at 0.5°C/min in oxygen to 350°C where it was maintained for one hour.

## EXAMPLE 38

The "non-acidic" nature of the catalyst of Example 37 was confirmed by its ability to aromatize n-heptane to toluene in high yield. At 538°C and 4 kPa (30 torr) heptane in nitrogen, toluene was forrmed in 83 - 88% selectivity at a conversion of 99 + %. Total yield of benzene plus toluene was greater than 90%.

## EXAMPLE 39

The catalyst of Example 37 was used to study the reforming of a hydrotreated Arab light naphtha, boiling range 82 - 121°C (180 - 250°F). The reaction was run at 538°C at atmospheric pressure at 1.8 WHSV abd a N$_2$/HC ratio of 2.2. The results obtained are shown below.

| | Feed | Product | % Converted |
|---|---|---|---|
| C$_1$-C$_4$ | 0 | 0.4 | |
| Methylpentanes | 16.5 | 11.6 | 30% |
| n-Hexane | 24.2 | 12.2 | 50% |
| Methylhexanes | 15.6 | 11.8 | 24% |
| n-Heptane | 17.1 | 7.2 | 58% |
| Benzene | 2.1 | 14.0 | |
| Toluene | 3.2 | 11.5 | |

The above results indicate highly selective aromatics formation together with very low C$_1$-C$_4$ gas production.

## EXAMPLE 40

Lead-containing ZSM-5 was synthesized. A solution A was prepared by dissolving 3.31 g Pb(NO$_3$)$_2$ in 338.8 g de-ionized water. A solution B was prepared by dissolving 12.4 g NaOH in 300 g de-ionized water. 23.94 TPA bromide was then dissolved in solution B, which was then poured into solution A. 60.0 g silica gel (SPEX Ind.) was placed in a 1-liter stainless steel autoclave. The solution was now tranferred to the autoclave, and the mixture was stirred for two minutes before sealing the autoclave. Stirring and heating were begun immediately. The composition of the hydrogel formed is described by the following mole ratios.

| SiO$_2$/Pb | H$_2$O/SiO$_2$ | OH-/SiO$_2$ | Na + /SiO$_2$ | TPA + /SiO$_2$ |
|---|---|---|---|---|
| 90 | 40 | 0.30 | 0.34 | 0.10 |

The zeolite crystrallization was carried out at 160°C with stirring at 400 rpm for 4 days. The product ZSM-5 analyzed for 7.96% C, 0.7% N, 0.97% Na, 4.0% Pb, 86.48% Ash, and 235 ppm Al$_2$O$_3$. Platinum incorporation was effected in a manner similar to that in Example 37.

**Claims**

1. A catalyst composition comprising a dehydrogenation metal, and non-acidic ZSM-5 containing indium, tin, thallium, or lead.

2. The composition of Claim 1 wherein the dehydrogenation metal comprises 0.1 to 20 weight percent of the composition and said indium, tin, thallium, or lead comprises 0.05 to 20 weight percent of the composition.

3. The composition of Claim 1 wherein the dehydrogenation metal is selected from a Group VIII metal, chromium and vanadium.

4. The composition of Claim 1, wherein the dehydrogenation metal is platinum.

5. The composition of Claim 5, wherein the ZSM-5 contains less than 0.1 wt% aluminum.

6. A process for dehydrogenating a hydrocarbon reactant having at least 2 carbon atoms, comprising contacting said reactant with the catalyst composition of Claim 1, and producing a product having the same number of carbon atoms as the reactant and having its original hydrogen content decreased by at least two hydrogen atoms.

7. The process of Claim 6 wherein the reactant has at least 6 carbon atoms and the reaction involves at least some dehydrocyclization.

8. The process of Claim 6 or Claim 7 wherein the reactant is a naphtha and the product has a higher octane number than the reactant.

**Patentansprüche**

1. Katalysatorzusammensetzung, die ein Dehydrierungsmetall und einen nichtsauren ZSM-5 umfaßt, der Indium, Zinn, Thallium oder Blei enthält.

2. Zusammensetzung nach Anspruch 1, worin das Dehydrierungsmetall 0,1 bis 20 Gew.-% der Zusammensetzung umfaßt und das Indium, Zinn, Thallium oder Blei 0,05 bis 20 Gew.-% der Zusammensetzung umfassen.

3. Zusammensetzung nach Anspruch 1, worin das Dehydrierungsmetall aus Metallen der Gruppe VIII, Chrom und Vanadium ausgewählt ist.

4. Zusammensetzung nach Anspruch 1, worin das Dehydrierungsmetall Platin ist.

5. Zusammensetzung nach Anspruch 5, worin der ZSM-5 weniger als 0,1 Gew.-% Aluminium enthält.

6. Verfahren zur Dehydrierung eines Kohlenwasserstoffreaktanten mit mindestens 2 Kohlenstoffatomen, das den Kontakt dieses Reaktanten mit der Katalysatorzusammensetzung nach Anspruch 1 umfaßt und ein Produkt erzeugt, daß die gleiche Anzahl von Kohlenstoffatomen wie der Reaktant hat und dessen ursprünglicher Wasserstoffgehalt um mindestens 2 Wasserstoffatome abgenommen hat.

7. Verfahren nach Anspruch 6, worin der Reaktant mindestens 6 Kohlenstoffatome aufweist und die Reaktion mindestens eine geringe Dehydrocyclisierung umfaßt.

8. Verfahren nach Anspruch 6 oder 7, worin der Reaktant Benzin ist und das Produkt eine höhere Octanzahl als der Reaktant hat.

**Revendications**

1. Une composition catalytique comprenant un métal de déshydrogénation, et une ZSM-5 non acide contenant de l'indium, de l'étain, du thallium ou du plomb.

**2.** La composition selon la revendication 1, dans laquelle le métal de déshydrogénation correspond à 0,1 à 20% en poids de la composition et ledit indium, étain, thallium ou plomb correspond à 0,05 à 20% en poids de la composition.

**3.** La composition selon la revendication 1, dans laquelle le métal de déshydrogénation est choisi parmi un métal du groupe VIII, le chrome et le vanadium.

**4.** La composition selon la revendication 1, dans laquelle le métal de déshydrogénation est le platine.

**5.** La composition selon la revendication 1, dans laquelle la ZSM-5 contient moins de 0,1% en poids d'aluminium.

**6.** Un procédé de déshydrogénation d'une charge hydrocarbure ayant au moins 2 atomes de carbone, comprenant la mise en contact de ladite charge avec la composition catalytique selon la revendication 1, et la production d'un produit ayant le même nombre d'atomes de carbone que la charge et ayant sa teneur en hydrogène originelle diminuée d'au moins 2 atomes d'hydrogène.

**7.** Le procédé selon la revendication 6, dans lequel la charge a au moins 6 atomes de carbone et la réaction implique au moins une déshydrocyclisation.

**8.** Le procédé selon la revendication 6 ou 7, dans lequel la charge et le produit a un nombre d'octane supérieur à celui de la charge.

FIG.1

2θ

FIG. 2

C6/C7   LSR  NAPHTHA

PT - INZSM-5

PT-CI/ALUMINA

C5+YIELD,WT PCT

C5+RON+O

REFORMING OVER PT/SN-ZSM-5

FIG. 3

BENZENE

TOLUENE

GC AREA PER CENT

WHSV=1.35

T=527

WHSV=1.0

T=538          T=550

N-HEXANE

HOURS ON STREAM

EP 0 393 099 B1